# EUROPÄISCHE PATENTANMELDUNG

(11) **EP 3 845 576 A1**
(43) Veröffentlichungstag der Anmeldung: **07.07.2021**
(21) Anmeldenummer: 20150329.9
(22) Anmeldetag: 06.01.2020
(51) Int. Cl.: C08G 18/02, C08G 18/75

(54) **VERFAHREN ZUR HERSTELLUNG VON ISOCYANURAT**

(71) Anmelder: Evonik Operations GmbH, 45128 Essen (DE)
(72) Erfinder: SPYROU, Emmanouil, 46514 Schermbeck (DE); LOESCH, Holger, 44627 Herne (DE); KREISCHER, Susanne, 45701 Herten (DE); DIESVELD, Andrea, 48712 Gescher (DE); THESING, Andrea, 48683 Ahaus (DE)
(74) Vertreter: Evonik Patent Association

(57) **Zusammenfassung**

Gegenstand der vorliegenden Erfindung ist ein Verfahren zur Herstellung von Isocyanurat aus Diisocyanat, bei dem i) vorder Reaktion eine Bestimmung des Peroxidgehaltes des einzusetzenden Diisocyanats erfolgt, und danach ii) a) im Falle, dass der ermittelte Peroxidgehalt größer als 10 mmol/kg ist, das Diisocyanat einer destillativen Aufreinigung unterzogen wird, bis der ermittelte Peroxidgehalt kleiner oder gleich 10 mmol/kg ist, oder b) im Falle, dass der ermittelte Peroxidgehalt kleiner oder gleich 10 mmol/kg ist, keine weitere Aktion erfolgt, und iii) anschließend destilliertes Diisocyanat zu a) und/oder unbehandeltes Diisocyanat zu b) zu Isocyanurat umgesetzt wird.

## Beschreibung

Die vorliegende Erfindung betrifft ein Verfahren zur Herstellung von Isocyanuraten aus Diisocyanaten.

Isocyanurate stellen wertvolle Ausgangsmaterialien für die Herstellung von Polyurethanlacken dar. Insofern sind Verfahren zu ihrer Herstellung ebenfalls von großem Interesse,

EP 0 082 987 A2 offenbart ein Verfahren zur Herstellung von Isocyanuraten aus Gemischen enthaltend MPDI und EBDI. Die Herstellung erfolgt innerhalb von 1 - 60 Minuten bei Temperaturen von 40 - 140 °C in Gegenwart eines Katalysators, bei dem es sich bevorzugt um ein quaternäres Ammoniumsalz einer organischen Säure handeln kann.

EP 1 170 283 A2 offenbart ein Verfahren zur Herstellung geruchsarmer und lagerstabiler Isocyanurate, bei dem IPDI innerhalb von 30 Sekunden bis 2 Stunden bei Temperaturen von 0 bis 200 °C in Gegenwart eines Katalysators, bei dem es sich um ein Ammoniumsalz einer Säure handeln kann, umgesetzt wird.

EP 1 273 603 A1 offenbart ein Verfahren zur Herstellung geruchsarmer und lagerstabiler Isocyanurate, bei dem IPDI innerhalb von 3 Minuten bis 3 Stunden bei Temperaturen von 0 - 160 °C in Gegenwart eines Katalysators mit mindestens einem quaternären Stickstoffatom auf Basis eines tricyclischen Diamins umgesetzt wird.

EP 1 454 933 A1 offenbart ein Verfahren zur Herstellung geruchsarmer und lagerstabiler Isocyanurate, bei dem IPDI innerhalb von 2 - 30 Minuten bei Temperaturen von 20 - 120 °C und in einem Druckbereich von 0,5 - 5 bar in Gegenwart eines Katalysators mit mindestens einem quaternären Stickstoffatom auf Basis eines tricyclischen Diamins umgesetzt wird und anschließend der Katalysator thermisch deaktiviert wird.

Die bekannten Katalysatoren einsetzenden Verfahren zur Herstellung von Isocyanuraten haben den Nachteil, zu Produkten mit zu hohen Farbzahlen zu führen. Es ist somit Aufgabe der vorliegenden Erfindung pro Umsetzungsgrad an Isocyanurat möglichst wenig Farbigkeit zu erzeugen.

Überraschenderweise wurde festgestellt, dass insbesondere in älteren Diisocyanatchargen Peroxidverunreinigungen vorliegen, die zu den erwähnten niedrigen Umsetzungsgraden und hohen Farbzahlen bei der Isocyanuratsynthese führen. Weiterhin wurde festgestellt, dass höhere Ausbeuten und geringere Farbzahlen resultieren, wenn in den eingesetzten Diisocyanatchargen ein Peroxidgehalt von kleiner als 10 mmol/kg vorliegt.

Gegenstand der vorliegenden Erfindung ist demnach ein Verfahren zur Herstellung von Isocyanurat aus Diisocyanat, bei dem
i) vor der Reaktion eine Bestimmung des Peroxidgehaltes des einzusetzenden Diisocyanats erfolgt, und danach
ii)
   a) im Falle, dass der ermittelte Peroxidgehalt größer als 10 mmol/kg ist, das Diisocyanat einer destillativen Aufreinigung unterzogen wird, bis der ermittelte Peroxidgehalt kleiner oder gleich 10 mmol/kg ist, oder
   b) im Falle, dass der ermittelte Peroxidgehalt kleiner oder gleich 10 mmol/kg ist, keine weitere Aktion erfolgt, und
iii) anschließend destilliertes Diisocyanat zu a) und/oder unbehandeltes Diisocyanat zu b) zu Isocyanurat umgesetzt wird.

Gegenstand der vorliegenden Erfindung ist ein Verfahren zur Herstellung von Isocyanurat aus Diisocyanat. Bei dem Edukt "Diisocyanat" kann es sich um ein einzelnes Diisocyanat oder ein Gemisch von Diisocyanaten handeln. Bevorzugt handelt es sich bei dem Edukt um genau ein Diisocyanat.

Bevorzugt handelt es sich bei mindestens einem der eingesetzten Diisocyanate um ein (cyclo)aliphatisches Diisocyanat, d. h. um ein Diisocyanat mit mindestens einer direkt an einen aliphatischen Ring gebundenen Isocyanatgruppe und ggf. einer weiteren aliphatisch gebundenen (d. h. an einem über einen Alkylenrest mit dem aliphatischen Ring verbundenen) Isocyanatgruppe. Weiter bevorzugt wird nur ein (cyclo)aliphatisches Diisocyanat eingesetzt. Ganz besonders bevorzugt handelt es sich bei mindestens einem der eingesetzten Diisocyanate um Isophorondiisocyanat (IPDI) oder 4,4'-Diisocyanatodicyclohexylmethan (H12MDI). Noch weiter bevorzugt wird Isophorondiisocyanat als einziges Diisocyanat eingesetzt. Wird Isophorondiisocyanat eingesetzt, ist unerheblich, ob es über das Harnstoffverfahren oder über das Phosgenverfahren erhalten wurde.

Bei dem Produkt "Isocyanurat" handelt es sich prinzipiell um isocyanuratgruppenhaltige Produktgemische umfassend kettenförmige und quervernetzte Polyisocyanatoisocyanurate, Triisocyanatomonoisocyanurate ("Trimere") und ggf. Vorstufen der Trimerbildung.

Bevorzugt handelt es sich bei dem isocyanuratgruppenhaltigen Produktgemisch um monomerhaltiges Trimer, das durch eine partielle Trimerisierung von Diisocyanat herstellbar ist. Bevorzugt ist das erfindungsgemäße Verfahren zur Herstellung von Isocyanurat somit ein Verfahren zur partiellen Trimerisierung von Diisocyanat, bei dem im Wesentlichen Triisocyanatomonoisocyanurate und Vorstufen von Isocyanuraten entstehen.

Vor der partiellen oder vollständigen Umsetzung von Diisocyanat zu Isocyanurat erfolgt eine Bestimmung des Peroxidgehaltes im einzusetzenden Diisocyanat. Der Peroxidgehalt wird bestimmt gemäß DIN EN ISO 27 107 und wird bestimmt in mmol/kg.

Unter "vor" der Umsetzung ist dabei bevorzugt ein Zeitfenster von 14 Tagen bis 5 Minuten vor der Vermischung von Edukt und Katalysator zu verstehen. Ganz besonders bevorzugt ist darunter ein Zeitpunkt von 2 Tagen vor der Umsetzung zu verstehen.

Wird nur ein Diisocyanat zur Herstellung von Isocyanurat eingesetzt, ist der Peroxidgehalt des einzusetzenden Diisocyanats der Gehalt an Peroxid in mmol bezogen auf die Gesamtmasse des einzusetzenden Diisocyanats in Kilogramm. Wird mehr als ein Diisocyanat zur Herstellung von Isocyanurat eingesetzt, ist der Peroxidgehalt des einzusetzenden Diisocyanats der Gesamtgehalt an Peroxid in mmol bezogen auf die Gesamtmasse aller einzusetzenden Diisocyanate in Kilogramm.

In dem Fall, dass der wie zuvor definiert ermittelte Peroxidgehalt kleiner oder gleich 10 mmol/kg ist, erfolgt keine weitere Aktion, da nennenswerte Nachteile durch die Anwesenheit von einer solchen Konzentration an Peroxid nicht zu erwarten sind. Das oder die Edukte können somit direkt zu Isocyanurat umgesetzt werden.

Ist der wie zuvor definiert ermittelte Peroxidgehalt jedoch größer als 10 mmol/kg, wird das Edukt Diisocyanat einer destillativen Aufreinigung unterzogen. Soll aus nur einem Diisocyanat Isocyanurat hergestellt werden, wird jede Charge des Diisocyanats, deren Peroxidgehalt größer 10 mmol/kg ist, destillativ gereinigt, bis der Peroxidgehalt jeder Charge kleiner oder gleich 10 mmol/kg beträgt. Soll aus mehrerer unterschiedlichen Diisocyanaten Isocyanurat hergestellt werden, wird jede Charge jedes Diisocyanats, deren Peroxidgehalt größer 10 mmol/kg ist, destillativ gereinigt, bis der Peroxidgehalt jeder Charge jedes Diisocyanates kleiner oder gleich 10 mmol/kg beträgt.

Bevorzugt wird die destillative Aufreinigung in geeigneten Destillationskollonnen oder Destillationsaggregaten, z. B. Kurzweg- oder Dünnschichtdestillationsapparaturen, bei geeigneten Drücken und Temperaturen in Abhängigkeit der Siedetemperatur der Diisocyanate durchgeführt. Die Mindestdestillationstemperatur sollte dabei bevorzugt 100 °C nicht unterschreiten.

Die Umsetzung von Diisocyanat zu Isocyanurat wird bevorzugt in Gegenwart mindestens eines Katalysators bei Temperaturen von 0 - 160 °C durchgeführt. Der Druck wird dabei nicht gesondert eingestellt und entspricht dem Umgebungsdruck, der nahe bei 1 bar liegt. Bevorzugte Reaktionstemperaturen betragen 40 - 140 °C und noch weiter bevorzugt 60 - 130 °C.

Bevorzugte Reaktionszeiten liegen zwischen 3 Minuten und drei Stunden.

Geeignete Katalysatoren können ausgewählt werden aus der Gruppe bestehend aus tertiären Aminen, Alkalimetallsalzen von Carbonsäuren, quartären Ammoniumsalzen, Aminosilanen und quartären Hydroxyalkylammoniumsalzen. Bevorzugte Katalysatoren sind N-(2-Hydroxypropyl)-N,N,N-trimethylammonium-2-ethylhexanoat (75 %-ig in Diethylenglykol als DABCO TMR erhältlich), oder OH-haltige quartäre Ammoniumverbindungen (erhältlich z. B. als EP BZ 7078 B von Evonik).

Bevorzugt wird der Katalysator eingesetzt in Mengen von 0,05 - 1,5 Gew.-%, weiter bevorzugt in Mengen von 0,1 - 0,8 Gew.-%, noch weiter bevorzugt 0,4 - 0,7 Gew.-%, bezogen auf die Masse an eingesetztem Diisocyanat.

Die Reaktion kann optional in Gegenwart mindestens eines Co-Katalysators, mindestens eines Lösemittels und/oder mindestens eines Hilfsstoffes durchgeführt werden.

Bevorzugte Co-Katalysatoren können ausgewählt werden aus der Gruppe bestehend aus OHfunktionalisierten Verbindungen und Mannich-Basen aus sekundären Aminen und Aldehyden oder Ketonen.

Wird ein Lösemittel eingesetzt, wird mit diesem zur Erzielung einer exakteren Dosierung und optimalen Durchmischung bevorzugt der Katalysator gelöst. Bevorzugt sind Lösemittel ausgewählt aus Wasser, niedermolekularen Alkoholen (insbesondere Methanol und Ethylenglykol) und niedermolekularen organischen Säuren (insbesondere Essigsäure oder Hexansäure).

Das erfindungsgemäße Verfahren kann sowohl batchweise als auch kontinuierlich durchgeführt werden. Bevorzugt wird es im Batchverfahren durchgeführt.

Ganz besonders bevorzugt wird das erfindungsgemäße Verfahren als ein Verfahren zur partiellen Trimerisierung von Diisocyanat durchgeführt, d. h. bei der Umsetzung von Diisocyanat zu Isocyanurattrimer wird ein Umsatz deutlich unterhalb von 100 % angestrebt (bestimmt über den Restgehalt an NCO-Gruppen), bevorzugt zwischen 20 und 80 %, weiter bevorzugt zwischen 25 und 60 %, noch weiter bevorzugt zwischen 30 und 45 %. Der Umsatz wird einfach über eine titrimetrische NCO-Zahl-Bestimmung in Anlehnung an DIN EN ISO 14896:2009-07 durchgeführt, d. h. eine Probemenge wird in einem nicht protischem Lösemittel (z. B. Aceton oder Ethylacetat) gelöst, dann wird ein Überschuss an Dibutylamin zugegeben und der nicht reagierte Anteil mit 0,1%iger Salzsäure zurücktitriert.

Bei einem solchen Verfahren zur partiellen Trimerisierung von Diisocyanat lässt man Disocyanat in Gegenwart des Katalysators, ggf. unter Verwendung von Lösemitteln und/oder Hilfsstoffen, bis zum Erreichen des gewünschten Umsatzes reagieren. Bricht die Reaktion bei der Erzielung des gewünschten Umsatzes nicht ab, kann sie durch Deaktivierung des Katalysators abgebrochen werden. Dies kann durch Zusatz eines Katalysatorinhibitors wie beispielsweise p-Toluolsulfonsäure, Chlorwasserstoff oder Dibutylphosphat erfolgen. Nachteil ist dabei jedoch eine ggf. unerwünschte Kontamination des entstandenen isocyanuratgruppenhaltigen Polyisocyanats mit dem Katalysatorinhibitor.

Überraschend wurde darüber hinaus festgestellt, dass im Falle von Peroxidgehalten zwischen 0,1 und 10 mmol/kg die entsprechenden Diisocyanate, insbesondere IPDI, direkt zu Produktgemischen einer partiellen Trimerisierung (bevorzugt umfassend monomeres IPDI, trimeres Isophoronisocyanurat und höhere Oligomere mit Isocyanuratstruktur mit guten Eigenschaften umgesetzt werden können, ohne dass ein Abbruch der Reaktion unter Verwendung eines nachteiligen Katalysatorinhibitors erforderlich wird.

Gegenstand der vorliegenden Erfindung ist damit auch ein Verfahren zur Herstellung von Isocyanurat aus Diisocyanat, bei dem
i) vor der Reaktion eine Bestimmung des Peroxidgehaltes des einzusetzenden Diisocyanats erfolgt, und danach
ii)
   a) im Falle, dass der ermittelte Peroxidgehalt größer als 10 mmol/kg ist, das Diisocyanat einer destillativen Aufreinigung unterzogen wird, bis der ermittelte Peroxidgehalt kleiner oder gleich 10 mmol/kg, aber größer oder gleich 0,1 mmol/kg ist,
      oder
      im Fall, dass der ermittelte Peroxidgehalt kleiner als 0,1 mmol/kg ist, Peroxid zugesetzt wird, bis der Peroxidgehalt größer oder gleich als 0,1 mmol/kg, aber kleiner oder gleich 10 mmol/kg ist oder
   b) im Falle, dass der ermittelte Peroxidgehalt kleiner oder gleich 10 mmol/kg, aber größer als 0,1 mmol/kg ist, keine weitere Aktion erfolgt, und
iii) anschließend destilliertes Diisocyanat zu a) und/oder unbehandeltes Diisocyanat zu b) zu Isocyanurat umgesetzt wird.

### Beispiele:

Jeweils 100 g Isophorondiisocyanat werden auf 100 °C aufgeheizt und mit 0,5 % eines Trimerisierungskatalysators versetzt (DABCO TMR, Air Products oder Vestanat EP BZ 7078 B, Evonik). Die Mischung heizt sich durch eine exotherme Reaktion auf eine Temperatur unterhalb von 160 °C auf und wird danach abgekühlt. Man bestimmt die Rest-NCO-Gehalt und damit den Umsatz sowie die Farbzahl.

Als Ausgangsstoff wird entweder Isophorondiisocyanat, erhalten nach dem Harnstoffverfahren (IDPI U) oder nach dem Phosgenverfahren (IPDI P) eingesetzt.

Folgende Beobachtungen werden gemacht: Je mehr Peroxid das IPDI enthält, desto geringer ist der Umsatz bzw. die Reaktivität. Die Farbe hängt zum einen mit dem Peroxidgehalt zusammen, zum anderen aber auch mit dem Umsatz. D. h. Peroxide führen immer zu geringerem Umsatz, meist auch zu höheren Farbzahlen. Dies gilt sowohl für IPDI (U) wie auch für IPDI (P) und ist auch für beide verwendeten Katalysatoren zutreffend.
a) IPDI U, Katalysator: 0,5 Gew.-% DABCO TMR

| Nr. | Peroxidgehalt | T_{Start} | T_{Ende} | NCO-Gehalt | Umsatz | Farbzahl Hazen/Gardner | Farbe/Umsatz [Hz/%] |
|---|---|---|---|---|---|---|---|
| 1 | < 0,1 | 100°C | 136,9°C | 30,60% | 38,40% | 228/1,0 | 6 |
| 2 | 19 | 100°C | 120,6°C | 32,40% | 28% | 747/4,0 | 27 |
| 3 | 32 | 100°C | 122,4°C | 32,90% | 25,90% | 730/3,9 | 28 |
| 4 | 32 | 100°C | 111,3°C | 32,50% | 28,00% | 915/4,4 | 33 |
| 5 | 56 | 100°C | 102°C | 35,20% | 13,80% | 390/2,3 | 28 |

b) IPDI P, Katalysator: 0,5 Gew.-% BZ 7078

| Nr. | Peroxidgehalt | T_{Start} | T_{Ende} | NCO-Gehalt | Umsatz | Farbzahl Hazen/Gardner | Farbe/Umsatz [Hz/%] |
|---|---|---|---|---|---|---|---|
| 1 | < 0,1 | 100°C | 134,7°C | 29,70% | 42,90% | 514/3,0 | 12 |
| 2 | 13,5 | 100°C | 116,2°C | 32,70% | 27,00% | 567/3,3 | 21 |
| 3 | 23 | 100°C | 101,4°C | 35,60% | 11,60% | 396/2,3 | 34 |

## Patentansprüche

1. Verfahren zur Herstellung von Isocyanurat aus Diisocyanat, bei dem
i) vor der Reaktion eine Bestimmung des Peroxidgehaltes des einzusetzenden Diisocyanats erfolgt, und danach
ii)
a) im Falle, dass der ermittelte Peroxidgehalt größer als 10 mmol/kg ist, das Diisocyanat einer destillativen Aufreinigung unterzogen wird, bis der ermittelte Peroxidgehalt kleiner oder gleich 10 mmol/kg ist, oder
b) im Falle, dass der ermittelte Peroxidgehalt kleiner oder gleich 10 mmol/kg ist, keine weitere Aktion erfolgt, und
iii) anschließend destilliertes Diisocyanat zu a) und/oder unbehandeltes Diisocyanat zu b) zu Isocyanurat umgesetzt wird.

2. Verfahren nach Anspruch 1,
**dadurch gekennzeichnet, dass**
das Verfahren ein Verfahren zur Herstellung von Isocyanurat aus einem Diisocyanat ist.

3. Verfahren nach Anspruch 1 oder 2,
**dadurch gekennzeichnet, dass**
mindestens eines der eingesetzten Diisocyanate ein (cyclo)aliphatisches Diisocyanat ist.

4. Verfahren nach einem der vorhergehenden Ansprüche,
**dadurch gekennzeichnet, dass**
mindestens eines der eingesetzten Diisocyanate Isophorondiisocyanat oder 4,4'-Diisocyanatodicyclohexylmethan ist.

5. Verfahren nach einem der vorhergehenden Ansprüche,
**dadurch gekennzeichnet, dass**
die Umsetzung von Diisocyanat zu Isocyanurat in Gegenwart mindestens eines Katalysators bei Temperaturen von 0 - 160 °C durchgeführt wird.

6. Verfahren nach Anspruch 5,
**dadurch gekennzeichnet, dass**
der Katalysator ausgewählt ist aus der Gruppe bestehend aus N-(2-Hydroxypropyl)-N,N,N-trimethylammonium-2-ethylhexanoat und OH-haltigen quartären Ammoniumverbindungen.

7. Verfahren nach einem der vorhergehenden Ansprüche,
**dadurch gekennzeichnet, dass**
der Umsatz von Diisocyanat zu Isocyanurattrimer zwischen 20 und 80 Gew.-% beträgt.

8. Verfahren nach einem der vorhergehenden Ansprüche,
**dadurch gekennzeichnet, dass**
in Schritt
ii)
a) im Falle, dass der ermittelte Peroxidgehalt größer als 10 mmol/kg ist, das Diisocyanat einer destillativen Aufreinigung unterzogen wird, bis der ermittelte Peroxidgehalt kleiner oder gleich 10 mmol/kg, aber größer oder gleich 0,1 mmol/kg ist,
oder
im Fall, dass der ermittelte Peroxidgehalt kleiner als 0,1 mmol/kg ist, Peroxid zugesetzt wird, bis der Peroxidgehalt größer oder gleich als 0,1 mmol/kg, aber kleiner oder gleich 10 mmol/kg ist oder
b) im Falle, dass der ermittelte Peroxidgehalt kleiner oder gleich 10 mmol/kg, aber größer als 0,1 mmol/kg ist, keine weitere Aktion erfolgt.
